# EUROPÄISCHE PATENTSCHRIFT

(11) **EP 3 228 349 B1**
(45) Veröffentlichungstag und Bekanntmachung des Hinweises auf die Patenterteilung: **24.10.2018**
(21) Anmeldenummer: 17162799.5
(22) Anmeldetag: 24.03.2017
(51) Int. Cl.: A61M 37/00

(54) **HUBEINSTELLVORRICHTUNG FÜR L-FÖRMIGE MIKRONADELVORRICHTUNG**
STROKE ADJUSTMENT DEVICE FOR L-SHAPED MICRONEEDLE DEVICE
DISPOSITIF DE RÉGLAGE DE COURSE POUR DISPOSITIF DE MICRO-AIGUILLE EN FORME DE L

(30) Priorität: 05.04.2016 DE 202016101783 U
(43) Veröffentlichungstag der Anmeldung: 11.10.2017
(73) Patentinhaber: Arnold, Werner, 69115 Heidelberg (DE)
(72) Erfinder: Arnold, Werner, 69115 Heidelberg (DE)
(74) Vertreter: Weber & Seidel

(56) Entgegenhaltungen:
- EP-A1- 2 944 349
- BE-A2- 859 223
- DE-U1- 29 701 929
- US-A- 6 065 371
- US-A1- 2014 324 089
- US-B1- 7 207 242

## Beschreibung

Die Erfindung betrifft eine Hubeinstellvorrichtung für eine L-förmige Mikronadelvorrichtung.

Mikronadelvorrichtungen dienen der physikalischen Hautbehandlung. Dabei werden feine, das heißt atraumatische, Nadeln von 0,1 bis ca. 0,25 mm Durchmesser in die obere Schicht der Haut, die Epidermis, eingestochen, um winzige Verletzungen zu erzeugen, die so gering sind, daß sie keine Narben bilden. Dies stimuliert die Hautregeneration, was eine Hautverbesserung bei Narben, Pigmentstörungen, Falten und ähnlichen Hautproblemen bewirkt. Durch die erzeugten Mikroverletzungen werden u.a. hauteigene Wachstumsfaktoren freigesetzt, Kollagenfasern und Blutgefäße neu gebildet und die Zuwanderung von Fibroblasten initiiert. Für die Erzeugung dieser Mikroverletzungen sind Nadelwalzen und Nadelstempel bekannt, bei denen eine Vielzahl von Nadeln manuell großflächig auf und in die Haut gebracht werden können. Mikronadelvorrichtungen können jedoch auch wie Tätowiervorrichtungen ausgestaltet sein, da auch diese dazu dienen, Nadeln in die Haut einzubringen. Dort wird jedoch gleichzeitig ein Farbstoff in die Haut eingebracht, der, wenn in die unter der Epidermis liegende Dermis eingebracht, eine dauerhafte Tätowierung ergibt, und wenn nur in die Epidermis eingebracht, ein mit der Zeit ausbleichendes Permanent-Makeup darstellt. Bei einer Mikronadelbehandlung werden die Nadeln ebenfalls nur in die Epidermis eingestochen, wobei die Einstechtiefe darüber bestimmt, ob es sich um eine kosmetische Behandlung (bis 0,5 mm) handelt oder um eine medizinische (über 0,5 mm).

Da die Haut an den verschiedenen Körperpartien eine unterschiedliche Dicke aufweist, ist die Einstichtiefe, das heißt der Überstand der Nadel vom Gehäuse bei der Vorschubbewegung, unabhängig von der Frage, ob die Vorrichtung zu medizinischen oder zu kosmetischen Behandlungen eingesetzt wird, von großer Bedeutung. Beispielsweise ist die Haut im Gesicht und auf dem Handrücken dünner als an anderen Körperpartien. Aber auch Narbengewebe kann eine dickere Hautstruktur bilden, so daß es wichtig ist, die Nadeleinstichtiefe variabel einstellen zu können, um sie an die unterschiedlich dicken Epidermisschichten anpassen zu können, die zwischen 0,03 mm und 2 mm variieren, im Gesichtsbereich mit besonders dünner Haut sogar nur zwischen 0,03 mm und 0,2 mm. Die Nadeleinstichtiefe bestimmt sich über den Überstand der Nadeln über das Gehäuse bei ausgefahrenem Hub.

Bei einer Mikronadelvorrichtung in L-Form wird die Rotationsbewegung aus einem querliegenden Antrieb in eine rechtwinklig dazu ausgerichtete Hubbewegung für den Nadelschaft umgesetzt. Die Kraftübertragung erfolgt üblicherweise mittels Exzenter, der an der Motorwelle läuft und einen Pleuel in Hubbewegung versetzt, die den Nadelschaft antreibt. Das Gehäuse ist üblicherweise zweiteilig ausgebildet, wobei der Griffteil mit dem darin verlaufenden Nadelschaft über eine endständige Bohrung im länglichen Motorgehäuseteil mit diesem verbunden ist, wobei der Nadelschaft durch diese Bohrung hindurch über ein Pleuel mit dem Antrieb gekoppelt ist. Am freien Ende des Griffteils befindet sich die Austrittsöffnung für die Nadel(n). Je nachdem, wie weit die Nadel(n) über diese Öffnung im ausgefahrenen Hub des Nadelschafts hinauskragen, bestimmt sich die Einstichtiefe, der Austritts- beziehungsweise der Stichhub. In dieser Anmeldung ist mit Hubeinstellung die Einstellung dieses Austritts- oder Stichhubs gemeint, der dem Überstand der Nadel(n) über der Nadelaustrittsöffnung entspricht.

Es sind Verstellvorrichtungen für die Nadeleinstichtiefe bei L-förmigen Mikronadelvorrichtungen bekannt, bei der der Griff-Gehäuseteil mehrteilig ausgebildet ist und ein Teil des Griff-Gehäuseteils gegenüber dem anderen Griff-Gehäuseteil durch Gewindeverbindung ein- und ausfahrbar ist. So ist beispielsweise aus der DE 297 01 929 U1 für eine Tätowiervorrichtung eine Einstellvorrichtung bekannt, die als endständige Justierhülse ausgebildet ist, die über ein Gewinde mit dem Griff verbunden und durch Drehung um den Griff herum entlang des Griffs axial verstellbar ist, um den Nadelüberstand zu verringern oder zu vergrößern. Bei der EP 2 944 349 A1 sitzt das Griffteil mittels Klemmverbindung am Kolbengehäuse und ist mittels einer Drehhülse auf dem Kolbengehäuse in verschiedenen Positionen fixierbar.

Nachteilig daran ist, daß die Verstellbarkeit während des Betriebs eingeschränkt ist, da das Stellelement im Bereich der Haltehand liegt und die Skala dadurch verdeckt wird. Außerdem birgt es wegen der Gewindelösung die Gefahr, daß die Einstellung während der Nutzung ungewollt und sogar unbemerkt verändert wird. Ein weiterer Nachteil ist, daß eine beabsichtigte Verstellung während des Betriebs nicht möglich ist.

Die US 2014/0 324 089 A1 schlägt Verstellmöglichkeiten des Stichhubs mittels verschiedener Linearantriebstypen oder durch Veränderung der Exzenterstellung vor. Die EP 2 954 925 A1 beschreibt einen Stellmechanismus, der auf die Exzenterstellung wirkt. Die EP 2 954 927 A1 offenbart einen Stellmechanismus, der auf die Kraftkopplungsposition entlang eines schräg ausgerichteten Exzenters wirkt. Diese Lösungen sind technisch aufwendig. Aufgabe der Erfindung ist daher, für eine L-förmige Mikronadelvorrichtung einen Verstellmechanismus nach dem Oberbegriff von Anspruch 1 für den Stichhub bereitzustellen, der die vorgenannten Nachteile überwindet.
Dies wird für eine Mikronadelvorrichtung der vorgenannten Art dadurch erreicht, daß der Griff-Gehäuseteil durch die Bohrung in den Motor-Gehäuseteil entlang eines Verstellwegs verschiebbar gelagert hineinkragt, wobei am zur Bohrung benachbarten Ende des Antriebs-Gehäuseteils eine Befestigungsöffnung ausgebildet ist, und Befestigungsmittel zur Verbindung des Griff-Gehäuseteils mit dem Motor-Gehäuseteil vorgesehen sind, die durch die Befestigungsöffnung mit dem Griff-Gehäuseteil so in Eingriff gebracht sind, daß sie den Griff-Gehäuseteil mittels Verstellmitteln verstellbar entlang eines Verstellwegs in einer definierten Position innerhalb des Motor-Gehäuseteils halten. Damit ist das Verstellelement aus dem Griffbereich heraus verlagert und ist dadurch im Betrieb immer im Blick. Eine versehentliche Verstellung kann ausgeschlossen werden. Gleichzeitig erlaubt es eine einfache Bedienung sogar während des Betriebs. Die Verstellmittel weisen dabei eine in den Befestigungsmitteln angeordnete Kurvenscheibe mit einer schneckenförmig von außen nach innen geführten Kurve in Form einer Aussparung sowie einen Kurvenstift auf, der auf dem Griff-Gehäuseteil so angeordnet ist, daß er in die Kurve eingreift, und daß die Kurvenscheibe in der Befestigungsöffnung entlang drehbar und der Kurvenstift während der Drehung in der Kurve führbar ist. Mit der Drehung der Kurvenscheibe wird der Kurvenstift entlang der Kurve von außen nach innen immer näher zur Mitte der Kurvenscheibe geführt, was bewirkt, daß der Griff-Gehäuseteil immer weiter in den Motor-Gehäuseteil hineinverschoben wird. Durch Drehung in die andere Richtung wird der Kurvenstift in der Kurvenscheibe wieder langsam von innen nach außen geführt, so daß der Griff-Gehäuseteil wieder aus dem Motor-Gehäuseteil ein Stück herausgeschoben wird. Das Maß der Kurvenkrümmung bestimmt dabei die Feinheit der Einstellbewegung. Durch die Führung innerhalb der schneckenförmigen Kurve kann eine fein dosierbare, stufenlose Verstellbarkeit erzielt werden. Die Kurve gibt dabei den maximalen Drehweg vor.

Eine vorteilhafte Weiterentwicklung sieht vor, daß in der Kurvenscheibe ein zentraler Anschlagstift angeordnet ist, und korrespondierend dazu im Griff-Gehäuseteil ein Langloch ausgebildet ist, in das der zentrale Anschlagstift eingreift, wobei das Langloch mit seiner Länge korrespondierend zum Verstellweg ausgerichtet ist, so daß der Anschlagstift im Betrieb während einer Verstellbewegung innerhalb des Langlochs geführt ist. Damit kann verhindert werden, daß sich der Griff-Gehäuseteil innerhalb der Bohrung im Motor-Gehäuseteil verdreht. Dies würde eine sichere Hubverstellung beeinträchtigen. Der zentrale Anschlagstift in der Kurvenscheibe wird von den Längsrändern des Langlochs während der Verstellbewegung geführt. Das Langloch muß dabei eine Länge aufweisen, die mindestens dem beabsichtigten Hubverstellweg entspricht. Bei entsprechender Dimensionierung kann das Langloch zusätzlich auch für eine weitere Begrenzung des Hubverstellwegs dienen.

Eine alternative Ausgestaltung sieht vor, daß anstelle der Bohrung eine rechtwinklige oder ovale Öffnung vorgesehen ist und der Griff-Gehäuseteil im Kontaktbereich mit dem Motor-Gehäuseteil einen korrespondierenden Querschnitt aufweist. Durch diese formschlüssige Führung kann eine Verdrehung des Griff-Gehäuseteils im Motor-Gehäuseteil ebenfalls verhindert werden.

Eine weitere vorteilhafte Weiterentwicklung sieht vor, daß die Befestigungsmittel eine Verstellkappe aufweisen, die so mit der Kurvenscheibe verbunden ist, daß die Kurvenscheibe durch die Befestigungsöffnung kragt und die Verstellkappe am Rand der Öffnung anschlägt, wobei die Kurvenscheibe mit der Verstellkappe drehfest verbunden ist. Dies erlaubt einen sicheren Verschluß der Öffnung.

Eine weitere vorteilhafte Weiterentwicklung sieht vor, daß die Verstellkappe im Kontaktbereich mit dem Rand der Befestigungsöffnung eine über einen Teil des Umfangs geführte Ausnehmung aufweist, die parallel zum Außenumfang der Verstellkappe verläuft und in der ein im Randbereich der Befestigungsöffnung angeordneter Anschlagstift führbar ist, so daß der Drehweg der Kurvenscheibe in der Befestigungsöffnung definiert begrenzt wird.

Eine zweckmäßige Weiterentwicklung sieht vor, daß die Verstellkappe eine Skala aufweist, die im Bereich des Drehwegs der Verstellkappe, der mit der Ausnehmung korrespondiert, das Maß des Hubverstellwegs anzeigt. Dadurch wird eine gezielte Einstellung ermöglicht. Eine vorteilhafte Ausgestaltung sieht vor, daß die Verstellkappe lösbar mit der Kurvenscheibe verbunden ist. Dies erlaubt eine gezielte Positionierung der Verstellkappe in der Null-Position der Kurvenscheibe.
Eine vorteilhafte Ausgestaltung sieht vor, daß die Kurvenscheibe ein Gewinde und der Motor-Gehäuseteil im Bereich seiner Befestigungsöffnung ein korrespondierendes Gewinde aufweist, so daß die Befestigungsmittel über Gewindeverbindung mit dem Motor-Gehäuseteil verbindbar sind. Dies erlaubt eine einfache und zuverlässige Montage.
Die Erfindung wird anhand der Zeichnung näher erläutert. Es zeigen.
- **Fig. 1**: eine Mikronadelvorrichtung in L-Form in Draufsicht nach dem Stand der Technik,
- **Fig. 2**: einen schematischen Ausschnitt mit der erfindungsgemäßen Hubeinstellvorrichtung teilgeschnitten in Draufsicht,
- **Fig. 3a, 3b, 3c**: Details der erfindungsgemäßen Hubeinstellvorrichtung zu Fig. 2 in Draufsicht und
- **Fig. 4**: weitere Details in Draufsicht in gleicher Blickrichtung wie in Fig. 3b.

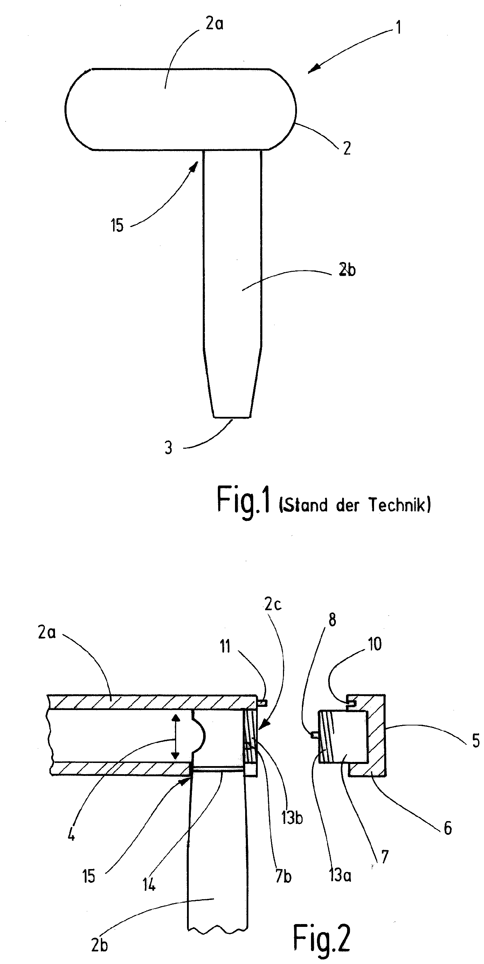

**Fig. 1** zeigt für eine Mikronadelvorrichtung 1 in L-Form nach Stand der Technik die Anordnung der Gehäuseteile 2a und 2b. Gehäuseteil 2a dient zur Aufnahme des Rotationsantriebs und Gehäuseteil 2b enthält den Hubantrieb, der den Nadelschaft antreibt, so daß die Nadeln durch die Nadelaustrittsöffnung 3 rhythmisch austreten können. Der Griff-Gehäuseteil 2b ist mittels einer Bohrung 15 im Motor-Gehäuseteil 2a mit diesem verbunden, um den Hubantrieb mit dem Rotationsantrieb zu koppeln, wobei jede Rotation im Motor-Gehäuseteil 2a zu einer Hubbewegung im Griffgehäuseteil 2b führt.

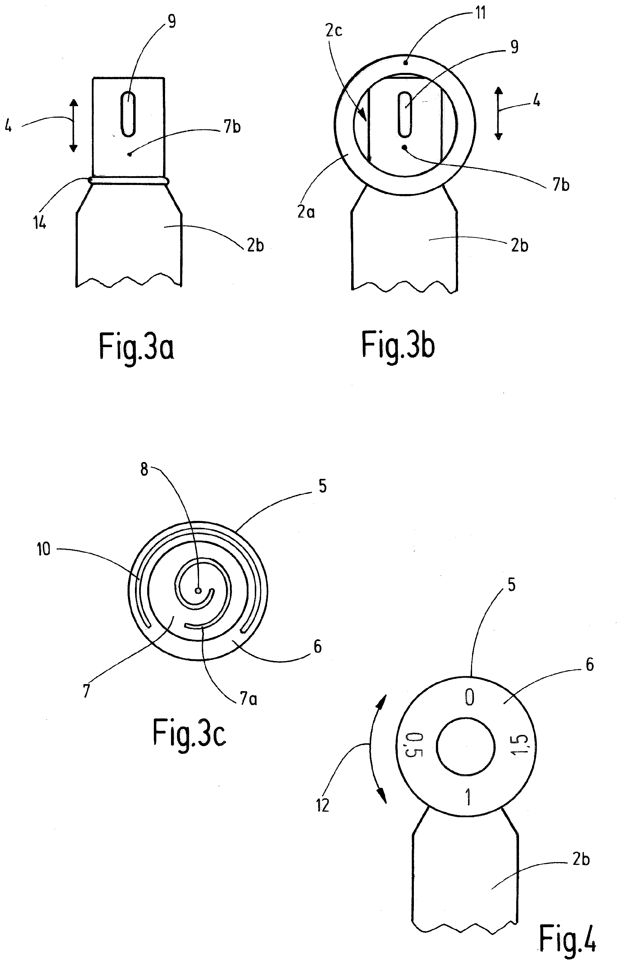

**Fig. 2** zeigt die erfindungsgemäße Elemente zur Verbindung der Gehäuseteile 2a, 2b mit den Befestigungsmitteln 5. Dabei ist das Motor-Gehäuseteil 2a längs aufgeschnitten dargestellt mit Blick auf das das durch die Bohrung 15 eingeführte Griff-Gehäuseteil 2b, das in diesem axial (in Bezug auf die Längsachse des Griff-Gehäuseteils 2b verschiebbar gelagert ist. Die Verschiebbarkeit ist durch Doppelpfeil 4 angedeutet, was auch dem Hubverstellweg entspricht. Auf dem Griff-Gehäuseteil 2b befindet sich ein O-Ring 14, der den Spalt zwischen dem Griff-Gehäuseteil 2b und dem Motor-Gehäuseteil 2a im Bereich der Bohrung abdichtet. Die Befestigungsmittel 5 sind separat dargestellt. Die außenliegende Verstellkappe 6 ist aufgeschnitten dargestellt, so daß im Profil die Ausnehmung 10 erkennbar ist, in die der im Randbereich der Befestigungsöffnung 2c des Motorgehäuseteils 2a angeordnete Anschlagstift 11 für die Verstellkappe 6 eingreift, um den Drehweg 12 (siehe Fig. 4) der Verstellkappe 6 zu begrenzen. Die Befestigungsmittel 5 umfassen ferner die in der Verstellkappe 6 angeordnete Kurvenscheibe 7, die in schematischer Draufsicht von der Seite dargestellt ist und die so weit aus der Verstellkappe 6 herauskragt, daß sie in die Befestigungsöffnung 2c einführbar ist. Auf der Kurvenscheibe 7 ist zentral der Anschlagstift 8 für die Verdrehsicherung. Der Anschlagstift 8 dient dazu, in das hier nicht dargestellte Langlochs 9 im Griff-Gehäuseteil 2b (siehe Fig. 3a) einzugreifen. Auf dem Griff-Gehäuseteil 2b ist der Kurvenstift 7b angeordnet, der in die hier nicht dargestellte Kurve 7a (siehe Fig. 3c) der Kurvenscheibe 7 eingreift, wenn das Befestigungsmittel die Gehäuseteile 2a und 2b verbindet. Gewinde 13a auf der Kurvenscheibe 7 und Gewinde 13b in der Befestigungsöffnung 2c dienen der lösbaren Verbindung der Befestigungsmittel 5 mit dem Motor-Gehäuseteil 2a. Gleichzeitig fixieren die Befestigungsmittel 5 im mit dem Motor-Gehäuseteil 2a verbundenen Zustand das Griff-Gehäuseteil 2b innerhalb des Motor-Gehäuseteils 2a derart, daß die Einschubposition des Griff-Gehäuseteils 2b innerhalb des Motor-Gehäuseteils 2a entlang des Hubverstellwegs 4 definiert verstellbar ist.

**Fig. 3a, 3b und 3c** zeigen Details zu den miteinander in Eingriff stehenden Elementen im Griff-Gehäuseteil 2b und in der Kurvenscheibe 7. **Fig. 3a** zeigt das Ende des Griff-Gehäuseteils 2b, das in das Motor-Gehäuseteil 2a eingeschoben werden kann, wobei der O-Ring 14 den Spalt zwischen dem Griff-Gehäuseteil 2b und dem Motor-Gehäuseteil 2a im Bereich der Bohrung abdichtet, durch die der Griff-Gehäuseteil 2b in den Motor-Gehäuseteil 2a eingeführt ist. Dargestellt ist das Langloch 9, in das der Anschlagstift 8 für die Verdrehsicherung eingreift, der zentral auf der Kurvenscheibe 7 angeordnet ist (siehe Fig. 3c). Dargestellt ist ferner der Kurvenstift 7b, der auf dem Griff-Gehäuseteil 2b angeordnet ist und in die Kurve 7a der Kurvenscheibe 7 eingreift.

**Fig. 3b** zeigt in Draufsicht das in den Motor-Gehäuseteil 2a eingeführte Griff-Gehäuseteil 2b, auf den man durch die Befestigungsöffnung 2c hineinschaut. Innerhalb der Befestigungsöffnung 2c erkennbar ist das Langloch 9 und der Kurvenstift 7b auf dem eingeschobenen Griff-Gehäuseteil 2b. Ferner dargestellt ist der Anschlagstift 11 für die Verstellkappe 6, der im Randbereich der Befestigungsöffnung 2c angeordnet ist und in die Ausnehmung 10 der Verstellkappe 6 (siehe Fig. 3c) eingreift. Doppelpfeil 4 deutet den Verstellweg des Griff-Gehäuseteils an. Der O-Ring 14 auf dem Griff-Gehäuseteil 2b ist hier nicht sichtbar, da er im Bereich der Gehäusewand vom Motor-Gehäuseteil 2a liegt und in dieser Darstellung verdeckt ist.

**Fig. 3c** zeigt in schematischer Draufsicht die korrespondierende Front der Befestigungsmittel 5 mit Verstellkappe 6 und darin angeordneter Kurvenscheibe 7 mit den Elementen, die mit dem Griff-Gehäuseteil 2b in Eingriff kommen: Zentral in der Kurvenscheibe 7 angeordnet ist der Anschlagstift 8 für die Verdrehsicherung, der in das Langloch 9 (siehe Fig. 3a, 3b) des Griff-Gehäuseteils 2b eingreift und dadurch verhindert, daß sich das Griff-Gehäuseteil 2b im Motor-Gehäuseteil 2a verdrehen kann. Im Randbereich der Verstellkappe 6 ist die Ausnehmung 10 dargestellt, in die der auf dem Randbereich der Befestigungsöffnung 2c angeordnete Anschlagstift 11 (siehe Fig. 3c) eingreift, um den Drehweg 12 (siehe Fig. 4) der Verstellkappe 6 zu begrenzen. Einer Drehung der Kurvenscheibe 7 in der Befestigungsöffnung 2c führt dazu, daß der in Fig. 3a dargestellte Kurvenstift 7b auf dem Griff-Gehäuseteil 2b in der Kurve 7a je nach Drehrichtung entweder nach innen oder nach außen geführt wird, so daß der Griff-Gehäuseteil 2b entlang des Verstellwegs 4 (Siehe Fig. 3a) verschoben wird. Das Maß des Verstellwegs wird durch den Krümmungsgrad der Kurve 7a bestimmt.

**Fig. 4** zeigt dieselbe Sicht mit angeordneten Befestigungsmitteln 5 in gleicher Blickrichtung wie bei Fig. 3b. Hier sind die Befestigungsmittel 5 in die Befestigungsöffnung 2c eingefügt, und die Verstellkappe 6 ist mit einer Skala versehen, die mit dem Verstellweg 12 der Kappe korrespondiert. Das richtig positionierte Aufsetzen in Nullstellung wird durch das nachträgliche Aufsetzen der Verstellkappe Kappe 6 auf die Kurvenscheibe 7 möglich, nachdem diese in Null-Position, das heißt auf dem niedrigsten Nadelaustriebshub, eingesetzt ist. Erst dann wird die Kappe 6 so mit der Kurvenscheibe 7 drehfest verbunden, so daß die Skala die Null-Position anzeigt. Die Drehfestigkeit der Verbindung zwischen Kurvenscheibe 7 und Kappe 6 kann beispielsweise mit Schrauben, beispielsweise in Form von Madenschrauben, oder mit anderen geeigneten Befestigungsmitteln vorgenommen werden. Ein Stift, der durch eine durch beide Elemente geführt Bohrung eingeführt wird, kann die drehfeste Verbindung sicherstellen. Ein solcher Stift und die vorgenannten Madenschrauben sind hier zwar nicht dargestellt, dienen aber trotzdem dazu, den Erfindungsgedanken weiter auszugestalten.

Die in den Figuren dargestellten Beispiel dienen lediglich dem besseren Verständnis des prinzipiellen Erfindungsgedankens und umfassen alle den Erfindungsgedanken verwirklichende Ausführungsformen. Beispielsweise kann die Verbindung der Verstellkappe mit der Kurvenscheibe auch anders als beschrieben ausgestaltet sein, nämlich indem die Kurvenscheibe von außen drehbar in der Kappe gelagert ist, die in dem Fall dann nicht zum Verstellen, sondern zur Verbindung der Verstellmittel mit dem Griff-Gehäuseteil dient. In dem Fall würde die Verstellung anhand der Drehung der Kurvenscheibe in der Kappe dienen, die drehfest mit dem Motor-Gehäuseteil in der Befestigungsöffnung angeordnet ist. Die Skala wäre in diesem Fall auf der Kurvenscheibe angeordnet, die sich an einem Kontrollpunkt auf der Kappe entlangbewegt. Bei dieser Ausgestaltung könnte die Befestigungsöffnung auch eine andere als runde Form aufweisen, beispielsweise rechteckig oder oval, so daß die Drehfestigkeit durch Formschluß zwischen Kappe und Befestigungsöffnung herstellbar ist.

### Bezugszeichenliste

- 1: Mikronadelvorrichtung
- 2: Gehäuse
- 2a: Motor-Gehäuseteil (zur Aufnahme des Rotationsantriebs)
- 2b: Griff-Gehäuseteil (zur Aufnahme des Hubantriebs mit dem Nadelschaft)
- 2c: Befestigungsöffnung (im Motor-Gehäuseteil)
- 3: Nadelöffnung
- 4: Doppelpfeil: Verschiebeweg, Hubverstellweg
- 5: Befestigungsmittel
- 6: Verstellkappe
- 7: Kurvenscheibe
- 7a: Kurve
- 7b: Kurvenstift
- 8: Anschlagststift für Verstellweg
- 9: Langloch
- 10: Ausnehmung
- 11: Anschlagstift für Verstellkappe
- 12: Doppelpfeil: Drehweg der Kappe
- 13a,13b: Gewinde
- 14: O-Ring
- 15: Bohrung (im Motor-Gehäuseteil)

## Patentansprüche

1. Hubeinstellvorrichtung für eine L-förmige Mikronadelvorrichtung (1), umfassend ein Gehäuse (2) mit einem querliegenden, länglichen Motor-Gehäuseteil (2a) zur Aufnahme des Rotationsantriebs und einem im rechten Winkel über eine endständige Bohrung (15) damit verbundenen röhrenförmigen Griff-Gehäuseteil (2b) zur Aufnahme des Hubantriebs für einen Nadelschaft,
**dadurch gekennzeichnet,**
**daß** der Griff-Gehäuseteil (2b) durch die Bohrung (15) in den Motor-Gehäuseteil (2a) entlang eines Verstellwegs (4) verschiebbar gelagert hineinkragt, wobei am zur Bohrung (15) benachbarten Ende des Antriebs-Gehäuseteils (2a) eine Befestigungsöffnung (2c) ausgebildet ist, und Befestigungsmittel (5) zur Verbindung des Griff-Gehäuseteils (2b) mit dem Motor-Gehäuseteil (2a) vorgesehen sind, die durch die Befestigungsöffnung (2c) mit dem Griff-Gehäuseteil (2b) so in Eingriff gebracht sind, daß sie den Griff-Gehäuseteil (2b) mittels Verstellmitteln (7, 7a, 7b) verstellbar entlang des Verstellwegs (4) in einer definierten Position innerhalb des Motor-Gehäuseteils (2a) halten, und daß die Verstellmittel (7, 7a, 7b) eine in den Befestigungsmitteln (5) angeordnete Kurvenscheibe (7) mit einer schneckenförmig von außen nach innen geführten Kurve (7a) in Form einer Aussparung (7a) aufweisen sowie einen Kurvenstift (7b), der auf dem Griff-Gehäuseteil (2b) so angeordnet ist, daß er in die Kurve (7a) eingreift, und daß die Kurvenscheibe (7) in der Befestigungsöffnung (2c) drehbar und der Kurvenstift (7b) während der Drehung in der Kurve (7a) führbar ist.

2. Hubeinstellvorrichtung nach Anspruch 1,
**dadurch gekennzeichnet,**
**daß** in der Kurvenscheibe (7) ein zentraler Anschlagstift (8) angeordnet ist, und korrespondierend dazu im Griff-Gehäuseteil (2b) ein Langloch (9) ausgebildet ist, in das der zentrale Anschlagstift (8) eingreift, wobei das Langloch (9) mit seiner Länge korrespondierend zum Verstellweg (4) ausgerichtet ist, so daß der Anschlagstift (8) im Betrieb während einer Verstellbewegung innerhalb des Langlochs (9) geführt ist.

3. Hubeinstellvorrichtung nach Anspruch 1,
**dadurch gekennzeichnet,**
**daß** anstelle der Bohrung (15) eine rechtwinklige oder ovale Öffnung vorgesehen ist und der Griff-Gehäuseteil (2b) im Kontaktbereich mit dem Motor-Gehäuseteil (2a) einen korrespondierenden Querschnitt aufweist.

4. Hubeinstellvorrichtung nach Anspruch 2 oder 3,
**dadurch gekennzeichnet,**
**daß** die Befestigungsmittel (5) eine Verstellkappe (6) aufweisen, die so mit der Kurvenscheibe (7) verbunden ist, daß die Kurvenscheibe (7) durch die Befestigungsöffnung (2c) kragt und die Verstellkappe (6) am Rand der Befestigungsöffnung (2c) anschlägt, wobei die Kurvenscheibe (7) mit der Verstellkappe (6) drehfest verbunden ist.

5. Hubeinstellvorrichtung nach Anspruch 4,
**dadurch gekennzeichnet,**
**daß** die Verstellkappe (6) im Kontaktbereich mit dem Rand der Befestigungsöffnung (2c) eine über einen Teil des Umfangs geführte Ausnehmung (10) aufweist, die parallel zum Außenumfang der Verstellkappe (6) verläuft und in der ein im Randbereich der runden Befestigungsöffnung (2c) angeordneter Anschlagstift (11) führbar ist, so daß der Drehweg (12) des Befestigungsmittels (5) in der Befestigungsöffnung (2c) definiert begrenzt wird.

6. Hubeinstellvorrichtung nach Anspruch 5,
**dadurch gekennzeichnet,**
**daß** die Verstellkappe (6) eine Skala aufweist, die im Bereich des Drehwegs (12) der Verstellkappe (6), der mit der Ausnehmung (10) korrespondiert, das Maß des Hubverstellwegs (4) anzeigt.

7. Hubeinstellvorrichtung nach einem der Ansprüche 4 bis 6,
**dadurch gekennzeichnet,**
**daß** die Verstellkappe (6) lösbar mit der Kurvenscheibe (7) verbunden ist.

8. Hubeinstellvorrichtung nach einem der vorhergehenden Ansprüche,
**dadurch gekennzeichnet,**
**daß** die Kurvenscheibe (7) ein Gewinde (13a) und der Motor-Gehäuseteil (2a) im Bereich seiner Befestigungsöffnung (2c) ein korrespondierendes Gewinde (13b) aufweist, so daß die Befestigungsmittel (5) über Gewindeverbindung (13a, 13b) mit dem Motor-Gehäuseteil (2a) verbindbar sind.

## Claims

1. A travel-adjusting device for an L-shaped microneedle device (1), comprising a housing (2) having a transverse longitudinal motor housing part (2a) for accommodating the rotational drive and a tubular handle housing part (2b), connected thereto at a right angle by means of a terminal hole (15), for accommodating the travel drive for a needle shaft, **characterized in that** the handle housing part (2b) protrudes through the hole (15) into the motor housing part (2a) in a displaceably-mounted manner along an adjustment path (4), wherein a fastening opening (2c) is constructed at the end of the drive housing part (2a) adjacent to the hole (15), and fastening means (5) are provided for connecting the handle housing part (2b) to the motor housing part (2a), which fastening means are brought into engagement with the handle housing part (2b) through the fastening opening (2c) in such a manner that the fastening means hold the handle housing part (2b) in a defined position inside the motor housing part (2a) in an adjustable manner along the adjustment path (4) by means of adjustment means (7, 7a, 7b), and that the adjustment means (7, 7a, 7b) have a cam plate (7) arranged in the fastening means (5) with a cam (7a) guided helically from the outside inwards in the form of a recess (7a) and also a cam pin (7b), which is arranged on the handle housing part (2b) in such a manner that the cam pin engages into the cam (7a) and that the cam plate (7) is rotatable in the fastening opening (2c) and the cam pin (7b) can be guided in the cam (7a) during the rotation.

2. The travel-adjusting device according to Claim 1, **characterized in that** a central stop pin (8) is arranged in the cam plate (7), and a slot (9) is constructed in a manner corresponding thereto in the handle housing part (2b), into which slot the central stop pin (8) engages, wherein the length of the slot (9) is aligned in a manner corresponding to the adjustment path (4), so that during operation, the stop pin (8) is guided inside the slot (9) during an adjustment movement.

3. The travel-adjusting device according to Claim 1, **characterized in that**, instead of the hole (15), a right-angled or oval opening is provided, and the handle housing part (2b) has a corresponding cross section in the contact region with the motor housing part (2a).

4. The travel-adjusting device according to Claim 2 or 3, **characterized in that** the fastening means (5) have an adjustment cap (6), which is connected to the cam plate (7) in such a manner that the cam plate (7) protrudes through the fastening opening (2c) and the adjustment cap (6) stops at the edge of the fastening opening (2c), wherein the cam plate (7) is connected to the adjustment cap (6) in a rotationally-fixed manner.

5. The travel-adjusting device according to Claim 4, **characterized in that** the adjustment cap (6) has a recess (10) guided over part of the circumference in the contact region with the edge of the fastening opening (2c), which recess runs parallel to the outer circumference of the adjustment cap (6) and a stop pin (11) arranged in the edge region of the round fastening opening (2c) can be guided in the recess, so that the rotational path (12) of the fastening means (5) is delimited in the fastening opening (2c) in a defined manner.

6. The travel-adjusting device according to Claim 5, **characterized in that** the adjustment cap (6) has a scale which displays the extent of the travel adjustment path (4) in the region of the rotational path (12) of the adjustment cap (6), which corresponds to the recess (10).

7. The travel-adjusting device according to one of Claims 4 to 6, **characterized in that** the adjustment cap (6) is connected to the cam plate (7) in a detachable manner.

8. The travel-adjusting device according to one of the preceding claims, **characterized in that** the cam plate (7) has a thread (13a) and the motor housing part (2a) has a corresponding thread (13b) in the region of the fastening opening (2c) thereof, so that the fastening means (5) can be connected to the motor housing part (2a) by means of a threaded connection (13a, 13b).

## Revendications

1. Système de réglage de course pour un dispositif à micro-aiguille en forme de L (1) comprenant un boîtier (2) avec une partie de boîtier Moteur transversale longitudinale (2a) pour loger la commande de rotation et une partie de boîtier Préhension (2b) tubulaire reliée de ce fait à angle droit par le biais d'un trou terminal (15) pour loger la commande de course pour une tige d'aiguille, **caractérisé en ce que** la partie de boîtier Préhension (2b) fait saillie à travers le trou (15) dans la partie de boîtier Moteur (2a) logée mobile le long d'une course de réglage (4), une ouverture de fixation (2c) étant constituée à l'extrémité voisine du trou (15) de la partie de boîtier d'entraînement (2a) et des moyens de fixation (5) étant prévus pour liaison de la partie de boîtier Préhension (2b) avec la partie de boîtier Moteur (2a) qui sont mis en prise par l'ouverture de fixation (2c) avec la partie de boîtier Préhension (2b) de telle manière qu'ils maintiennent de façon réglable le long de la course de réglage (4) la partie de boîtier Préhension (2b) au moyen de moyens de réglage (7, 7a, 7b) dans une position définie à l'intérieur de la partie de boîtier Moteur (2a) et **en ce que** les moyens de réglage (7, 7a, 7b) comportent une came (7) disposée dans les moyens de fixation (5) avec une courbe (7a) passant hélicoïdalement de l'extérieur vers l'intérieur sous la forme d'un évidement (7a), ainsi qu'une tige de came (7b) qui est disposée sur la partie de boîtier Préhension (2b) de telle sorte qu'elle vient en prise dans la courbe (7a) et **en ce que** la came (7) peut tourner dans l'ouverture de fixation (2c) et la tige de came (7b) être guidée dans la courbe (7a) pendant la rotation.

2. Système de réglage de course selon la revendication 1, **caractérisé en ce qu'**une tige de butée (8) est disposée dans la came (7) et qu'un trou oblong (9) est constitué en correspondance à cela dans la partie de boîtier Préhension (2b) dans lequel vient en prise la tige butée centrale (8), le trou oblong (9) étant orienté dans sa longueur en correspondance avec la course de réglage (4) de telle manière que la tige de butée (8) est guidée en fonctionnement pendant un mouvement de réglage à l'intérieur du trou oblong (9).

3. Système de réglage de course selon la revendication 1, **caractérisé en ce qu'**à la place du trou (15) une ouverture rectangulaire ou ovale est prévue et la partie de boîtier Préhension (2b) comporte une section transversale correspondante dans la zone de contact avec la partie de boîtier Moteur (2a).

4. Système de réglage de course selon la revendication 2 ou 3, **caractérisé en ce que** les moyens de fixation (5) comportent un capuchon de réglage (6) qui est relié à la came (7), **en ce que** la came (7) dépasse à travers l'ouverture de fixation (2c) et le capuchon de réglage (6) vient en butée au bord de l'ouverture de fixation (2c), la came (7) étant reliée solidaire en rotation au capuchon de réglage (6).

5. Système de réglage de course selon la revendication 4, **caractérisé en ce que** le capuchon de réglage (6) dans la zone de contact avec le bord de l'ouverture de fixation (2c) comporte un évidement (10) passant sur une partie de la périphérie, qui s'étend parallèlement à la périphérie extérieure du capuchon de réglage (6) et dans lequel peut être guidée une tige de butée (11) disposée dans le bord de l'ouverture de fixation ronde (2c) de telle manière que la course rotationnelle (12) du moyen de fixation (5) est limitée de façon définie dans l'ouverture de fixation (2c).

6. Système de réglage de course selon la revendication 5, **caractérisé en ce que** le capuchon de réglage (6) comporte une échelle qui indique la mesure de la distance de réglage de course (4) dans la zone de la course rotationnelle (12) du capuchon de réglage (6) correspondant à l'évidement (10).

7. Système de réglage de course selon l'une quelconque des revendications 4 à 6, **caractérisé en ce que** le capuchon de réglage (6) est relié de façon amovible à la came (7).

8. Système de réglage de course selon l'une quelconque des revendications précédentes, **caractérisé en ce que** la came (7) comporte un filetage (13a) et la partie de boîtier Moteur (2a) un filetage correspondant (13b) dans la zone de son ouverture de fixation (2c) de telle manière que les moyens de fixation (5) peuvent être reliés par le biais d'un raccord fileté (13a, 13b) à la partie de boîtier Moteur (2a).
